# EUROPEAN PATENT APPLICATION

(11) **EP 3 725 268 A1**
(43) Date of publication of application: **21.10.2020**
(21) Application number: 19189269.4
(22) Date of filing: 31.07.2019
(51) Int. Cl.: A61F 2/24, A61F 2/966

(54) **CONNECTION RELEASE STRUCTURE AND SYSTEM THEREOF**

(30) Priority: 16.04.2019 CN 201910304565; 16.05.2019 CN 201910405371
(71) Applicant: Lepu Medical Technology (Beijing) Co., Ltd., Changping Tech. Zone Beijing 102200 (CN)
(72) Inventor: QIU, Kejin, Beijing, 102200 (CN); WU, Yongjian, Beijing, 102200 (CN); ZHAO, Xuancheng, Beijing, 102200 (CN); LIU, Pengfei, Beijing, 102200 (CN); CHANG, Rencao, Beijing, 102200 (CN); LIU, Xiaojian, Beijing, 102200 (CN); ZHANG, Yuxin, Beijing, 102200 (CN)
(74) Representative: Baudler, Ron

(57) **Abstract**

The invention discloses a connection release structure and a system, wherein the connection release structure is configured for connecting a release object and is arranged on a loading sheath tube, and comprises a connection fixing system and an unlocking sleeve system; the connection fixing system comprises a hook structure, the unlocking sleeve system comprises an unlocking sleeve, the connection fixing structure and the unlocking sleeve are movably connected with the loading sheath tube, the connection fixing structure is configured for being connected with the release object, and the unlocking sleeve is configured for locking the connection fixing structure and the release. The connection release structure and the connection fixing system provided by the invention can accurately control the stability and coaxiality of the valve stent in the release process, effectively adjust the position of the valve stent in the implantation process or completely retract the valve stent so as to ensure that the valve has a good working state after being implanted.

## Description

### FIELD OF THE INVENTION

The invention relates to the technical field of medical instruments, in particular to a connection release structure for implanting a prosthetic valve stent into a channel in a mammal body and a system thereof.

### BACKGROUND OF THE INVENTION

With the extended average lifespan and more serious aging trend of the global population, the incidence of valvular heart disease is significantly increased. Among diseases of the elderly patients, only inferior to hypertension and coronary heart disease, the valvular heart disease ranks third, with the incidence up to 13.3%. At the beginning, aortic valve disease can only be treated conservatively by drugs, and in the late 20th century, surgical aortic valve replacement (SAVR) has appeared, in which a prosthetic valve is implanted into the body by surgical thoracotomy, so that it has certain requirements on the physical conditions of patients. For elderly patients with high surgical risk, the SAVR treatment is listed as a contraindication. With the development of medical device technology, transcatheter aortic valve replacement (TAVR) began to be used for surgical treatment of high-risk patients in 2002, which advantages include no need for thoracotomy, small trauma and rapid recovery. Since the TAVR technique has been applied in the field of valve diseases, which brings great benefits to the elderly and high-risk patients who are contraindicated by surgical operation, and has achieved good results. More and more attention is paid to the research and treatment field of cardiovascular diseases, and a large number of senile degenerative valvular disease patients with high risk of surgical operation urgently need the effective minimally invasive treatment method. Over the next decade, a large number of new devices for TAVR valve products have emerged worldwide, and with continuous research and development innovation and product iterative updating, more than 10 TAVR valves have been approved for use in the market and have been implanted in more than 400,000 patients.

At present, there is a mechanical locking valve which can be completely retrievable in the market, the valve stent of the mechanical locking valve is weaved by nickel-titanium wires, and then the mechanical locking valve is locked by using a unique locking/unlocking device. The mechanical locking valve has the advantages that the working state of the valve can be observed before release is finished, and if the implantation condition is not ideal or the working state is not good, the mechanical locking valve can be completely retracted into a sheath to be implanted again or replaced. However, the disadvantage is that the mechanical locking is complex and axial locking, with too much compression on the valve annulus and left ventricular outflow tract, which is very easy to cause conduction block.

### SUMMARY OF THE INVENTION

It is an object of the present invention to overcome the disadvantages of the prior art, thereby providing a connection release structure and system that improves the safety and reliability of the release object implanting.

In a first aspect, there is provided a connection release structure for connecting a release object comprising a connection fixing system and an unlocking sleeve system, wherein the connection fixing system comprises a connection fixing structure movably connected with a loading sheath tube, the unlocking sleeve system comprises an unlocking sleeve movably connected with the loading sheath tube, and when the connection release structure is configured for being matched with a release object, the connection fixing structure is configured for being connected with the release object, and the unlocking sleeve is configured for fixing or unlocking the connection fixing structure and the release object.

In one of the embodiments, the connection fixing structure may be one or more of a hook structure, a clamp structure or a lasso structure;

In one of the embodiments, a side of the connection fixing structure close to the loading sheath tube is a proximal side, and a side thereof away from the loading sheath tube is a distal side;

In one of the embodiments, the clamp structure has a disconnected state and a clamped state;

In one of the embodiments, the clamp structure comprises a first clamping body and a second clamping body, the proximal side of the first clamping body is fixedly connected with the proximal side of the second clamping body, and the first clamping body and the second clamping body are configured for clamping and connecting a release object; preferably, when the clamp structure is in the clamped state, the distal side of the first clamping body abuts against the distal side of the second clamping body, and when the clamp structure is in the disconnected state, the distal side of the first clamping body is disconnected from the distal side of the second clamping body;

In one of the embodiments, the lasso structure has a contracted state and an expanded state;

In one of the embodiments, the connection fixing structure is configured to release or capture a release object.

In one of the embodiments, in a stowed state, the connection release structure is configured to partially or completely retracte the release object into the loading sheath tube;

In one of the embodiments, in the stowed state, the connection release structure is partially or completely retracted into the loading sheath tube;

In one of the embodiments, in the stowed state, the connection fixing system and the unlocking sleeve system are partially or completely retracted into the loading sheath tube;

In one of the embodiments, in the stowed state, the connection fixing system and the unlocking sleeve system are partially or completely retracted into the loading sheath tube.

In one of the embodiments, in the stowed state, the connection fixing structure and the unlocking sleeve are partially or completely retracted into the loading sheath tube.

In one of the embodiments, in a first state, the connection fixing structure is configured for being connected with a release object; in a second state, the connection fixing structure is configured for being disconnected from the release object;

In one of the embodiments, in the first state, the unlocking sleeve is configured for being sleeved on the joint of the connection fixing structure and the release object; or in the first state, the unlocking sleeve is close to the joint of the connection fixing structure and the release object.

In one of the embodiments, in the second state, the unlocking sleeve is not located at the joint of the connection fixing structure and the release object.

In one of the embodiments, in the second state, the unlocking sleeve is moved away from the joint of the connection fixing structure and the release object.

In one of the embodiments, in the stowed state, the connection fixing system and the unlocking sleeve system are partially or completely retracted into the loading sheath tube.

In one of the embodiments, in the stowed state, the connection fixing structure and the unlocking sleeve are partially or completely retracted into the loading sheath tube.

In one of the embodiments, the unlocking sleeve system comprises an outer tube arranged on a loading sheath tube, the outer tube is movably connected with the loading sheath tube, the tail end of the outer tube is provided with a second finger-shaped connecting rod, and the unlocking sleeve is arranged on the second finger-shaped connecting rod;

In one of the embodiments, the outer tube is buckling, magnetically, rotationally and/or slidably, most preferably, slidably connected with the loading sheath tube.

In one of the embodiments, the connection fixing system comprises an inner tube arranged on a loading sheath tube, the inner tube is movably connected with the loading sheath tube, the tail end of the inner tube is provided with a first finger-shaped connecting rod, and the connection fixing structure is arranged on the first finger-shaped connecting rod in a one-to-one correspondence mode; In one of the embodiments, the clamp structure comprises a first clamping body and a second clamping body, the proximal side of the first clamping body is connected with a first finger-shaped connecting rod, and the proximal side of the second clamping body is connected with the first finger-shaped connecting rod; In one of the embodiments, in the first state, the distal side of the first clamping body abuts against the distal side of the second clamping body; In one of the embodiments, in the second state, the distal side of the first clamping body is disconnected from the distal side of the second clamping body;

In one of the embodiments, the inner tube is buckling, magnetically, rotationally and/or slidably, further preferably, slidably connected with the loading sheath tube;

In one of the embodiments, the unlocking sleeve passes through the first finger-shaped connecting rods in a one-to-one correspondence mode;

In one of the embodiments, the inner tube is slidably connected with the outer tube;

In one of the embodiments, the sliding direction of the outer tube is the same as that of the inner tube;

In one of the embodiments, the loading sheath tube is provided with a first through hole matched with the outer tube;

In one of the embodiments, the outer tube is internally provided with a second through hole matched with the inner tube, and the extending direction of the second through hole is the same as that of the first through hole;

In one of the embodiments, the first through hole and the second through hole are coaxially arranged;

In one of the embodiments, the connection fixing structure and the unlocking sleeve are equally arranged, and both are 3-18, preferably, 3-6, and most preferably, 3, or preferably, 9-15, and most preferably, 12.

In a second aspect, there is provided a connection release system comprising a connection fixing system, an unlocking sleeve system and a release object, the connection fixing system comprises a connection fixing structure movably connected with a loading sheath tube, the unlocking sleeve system comprises an unlocking sleeve movably connected with the loading sheath tube, the connection fixing structure is connected with the release object, the unlocking sleeve locks the connection fixing structure and the release object.

In one of the embodiments, in a stowed state, the release object is partially or completely retracted into the loading sheath tube;

In one of the embodiments, in the stowed state, the connection release structure is partially or completely retracted into the loading sheath tube; more preferably, in the stowed state, the connection fixing system and the unlocking sleeve system are partially or completely retracted into the loading sheath tube.

In one of the embodiments, the connection fixing structure is configured to release or capture a release object.

In one of the embodiments, in a first state, the connection fixing structure is connected with a release object; in a second state, the connection fixing structure is disconnected from the release object;

In one of the embodiments, the cross-section of the unlocking sleeve is rectangular, circular or elliptical.

In one of the embodiments, in the stowed state, the connection fixing system and the unlocking sleeve system are partially or completely retracted into the loading sheath tube.

In one of the embodiments, in the stowed state, the connection fixing structure and the unlocking sleeve are partially or completely retracted into the loading sheath tube.

In one of the embodiments, the release object is a self-expanding valve stent comprising a valve stent body;

In one of the embodiments, the self-expanding valve stent further comprises a lug structure matched with the connection release system, and at least one end of the valve stent body is provided with the lug structure;

In one of the embodiments, a side of the connection fixing structure close to the loading sheath tube is a proximal side, and a side thereof away from the loading sheath tube is a distal side;

In one of the embodiments, the valve stent body is a mesh tubular structure, and the length of the valve stent body is less than the diameter of the valve stent body;

In one of the embodiments, the length of the valve stent body is 18 mm to 30 mm, and the diameter of the valve stent body is 18 mm to 35 mm;

In one of the embodiments, part or all of the connection fixing structure is a hook structure, the lug structure and the hook structure are arranged as a snap fastener, the hook structure is Ω-shaped, L-shaped, E-shaped, C-shaped or sawtooth-shaped, and the shape of the lug structure sequentially corresponds to T-shaped, L-shaped, E-shaped, C-shaped or sawtooth-shaped;

In one of the embodiments, in the second state, the unlocking sleeve is not located at the joint of the hook structure and the release object;

In one of the embodiments, in the second state, the unlocking sleeve leaves the joint of the hook structure and the release object;

In one of the embodiments, the length of the unlocking sleeve is greater than or equal to the length of the joint of the hook structure and the release object;

In one of the embodiments, the unlocking sleeve is matched or in clearance fit with the release object and the hook structure;

In one of the embodiments, in the first state, the unlocking sleeve is sleeved on the joint of the connection fixing structure and the release object; In one of the embodiments, in the first state, the unlocking sleeve is sleeved on the joint of the hook structure and the release object, and the unlocking sleeve is matched or in clearance fit with the release object and the hook structure;

In one of the embodiments, part or all of the connection fixing structures are lasso structures.

In one of the embodiments, the lasso structure has a contracted state and an expanded state;

In one of the embodiments, in the first state, the lug structure passes through a lasso structure, the lasso structure is in the contracted state, and the lasso structure and the lug structure are mutually clamped;

In one of the embodiments, in the first state, the unlocking sleeve is close to the joint of the lasso structure and the lug structure;

In one of the embodiments, the lasso structure is caused to be in the contracted state;

In one of the embodiments, in the second state, the unlocking sleeve is away from the joint of the lasso structure and the lug structure, causing the lasso structure to be in the expanded state;

In one of the embodiments, the distal side of the lasso structure extends radially;

In one of the embodiments, the lug structure is T-shaped, L-shaped, E-shaped, C-shaped or sawtooth-shaped;

In one of the embodiments, part or all of the connection fixing structure is a clamp structure comprising a first clamping body and a second clamping body, and the proximal side of the first clamping body is fixedly connected with the proximal side of the second clamping body;

In one of the embodiments, in the first state, the first clamping body and second clamping body clamp a part of the valve stent body between the first clamping body and second clamping body.

In one of the embodiments, in the first state, a first cavity is formed between the first clamping body and the second clamping body, and a part of the valve stent body is positioned in the first cavity;

In one of the embodiments, in the second state, the first clamping body is disconnected from the second clamping body;

In one of the embodiments, in the second state, the distal side of the first clamping body is separated from the distal side of the second clamping body.

In a third aspect, there is provided a connection release system for a medical implant retracted into a loading sheath tube, comprising a hook system for connecting the medical implant and an unlocking sleeve system for locking or releasing the hook system from the medical implant, the hook system and the unlocking sleeve system being relatively slidably disposed within the loading sheath tube from inside to outside, wherein the hook system has a hook structure for capturing the medical implant, the unlocking sleeve system is provided with an unlocking sleeve, and after the hook structure is engaged with the medical implant, the unlocking sleeve slides to a junction to be locked or slides away from the junction to be released under the action of external force;

Preferably, the hook system comprises an inner tube coaxially arranged with the loading sheath tube, the tail end of the inner tube is provided with a first finger-shaped connecting rod, and the hook structures is arranged on the finger-shaped connecting rod in a one-to-one correspondence mode;

Preferably, the unlocking sleeve system comprises an outer tube sleeved outside the inner tube, the tail end of the outer tube is provided with a second finger-shaped connecting rod which is not fewer than the first finger-shaped connecting rod, and the unlocking sleeve passes through the first finger-shaped connecting rod in a one-to-one correspondence mode and is arranged on the second finger-shaped connecting rod;

Preferably, the hook structure and the unlocking sleeve are equally arranged, and both are 3-18.

The above connecting and releasing structure and the system thereof are retracted into the loading sheath tube in the stowed state and comprise a connection fixing system and an unlocking sleeve system, the connection fixing system comprises a connection fixing structure, the unlocking sleeve system comprises an unlocking sleeve, the connection fixing structure and the unlocking sleeve are slidably arranged on the loading sheath tube, the connection fixing structure is configured for being connected with a release object, and the unlocking sleeve is configured for locking the connection fixing structure and the release object. By adopting the scheme, the release object is connected with the connection fixing system and locked by the unlocking sleeve in the delivering process, and after the release object and the connection fixing system reach the target position, the loading sheath tube, the release object, the connection fixing system and the unlocking sleeve system are moved towards the proximal end to be transformed in an expansion state and supported to the target position; in the locking state of the unlocking sleeve, if the implantation site or the implantation state is found to be not ideal, a part or all of the release object, the connection fixing system and the unlocking sleeve system can be re-stowed(re-retracted) in the loading sheath tube by moving the loading sheath tube towards the distal end, and the release object, the connection fixing system and the unlocking sleeve system can be re-adjusted and released or the whole system can be withdrawn out of the body, so that a fault-tolerant opportunity is provided for an operator, and the safety and reliability of the release object implantation are improved.

### BRIEF DESCRIPTION OF THE DRAWINGS

In order to more clearly illustrate the embodiments of the invention or technical solutions of the prior art, the drawings required for use in the embodiment or prior art description follows will now be briefly described. The drawings in the following description are only some embodiments of the present invention, and those skilled in the art can obtain other drawings according to the drawings without any creative work.
FIG. 1 is a structurally schematic view showing a state in which a connection release system is connected with a release object according to one embodiment of the connection release system of the present invention;
FIG. 2 is a structurally schematic view showing a state in which a connection release system is locked with a release object according to one embodiment of the connection release system of the present invention;
FIG. 3 is a structurally schematic view of a connection fixing structure (hook structure) according to one embodiment of the connection release system of the present invention;
FIG. 4 is a schematic diagram of an unlocking sleeve system according to one embodiment of the connection release system of the present invention;
FIG. 5 is a structurally schematic view of a valve stent according to an embodiment of the present invention;
FIG. 6 is a structurally schematic view of a valve stent according to another embodiment of the present invention;
FIG. 7 is a schematic view showing a connection form of a lug structure and a hook structure in the present invention;
FIG. 8 is a schematic view showing another connection form of the lug structure and the hook structure in the present invention;
FIG. 9 is a schematic view showing yet another connection form of the lug structure and the hook structure in the present invention;
FIG. 10 is a schematic view showing yet another connection form of the lug structure and the hook structure in the present invention;
FIG. 11 is a schematic view showing yet another connection form of the lug structure and the hook structure in the present invention;

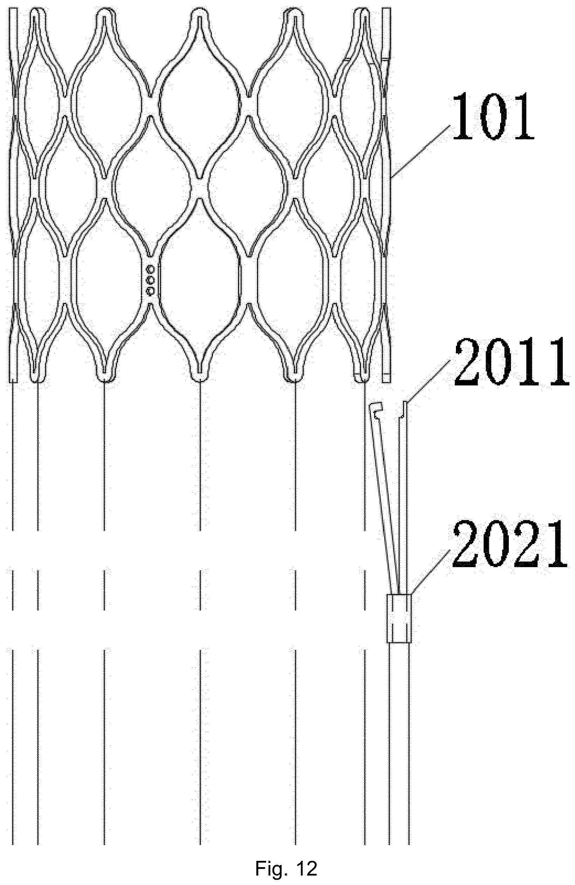

FIG. 12 is a schematic view (not clamped) of a connection form of the clamp structure and the valve stent body in the present invention;

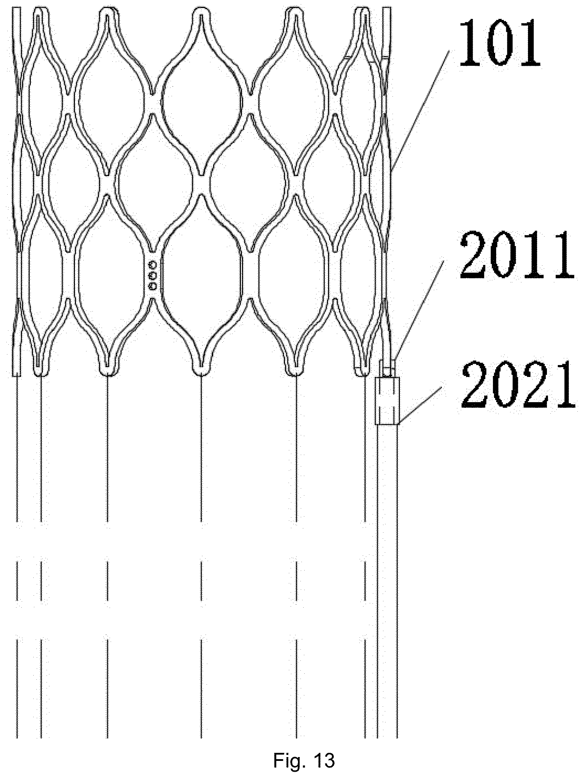

FIG. 13 is a schematic view (clamped) of a connection form of the clamp structure and the valve stent body in the present invention;

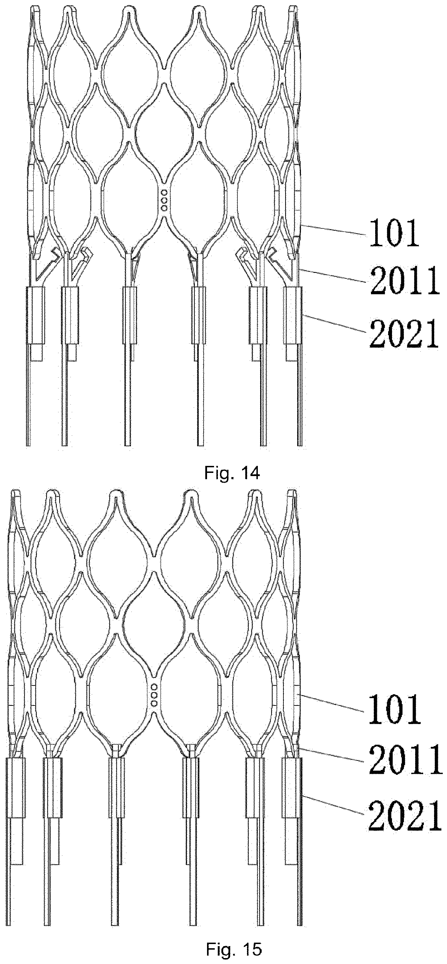

FIG. 14 is a schematic view (not clamped) of a connection form of the clamp structure and the valve stent body in the present invention;
FIG. 15 is a schematic illustration (clamped) of a connection form of the clamp structure and the valve stent body in the present invention;

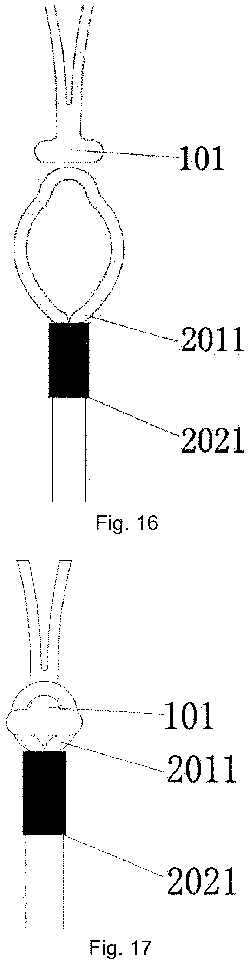

FIG. 16 is a partial schematic view (expanded state) of a connection form of the lasso structure and the lug structure in the present invention;
FIG. 17 is a partial schematic view (contracted state) of a connection form of the lasso structure and the lug structure in the present invention;

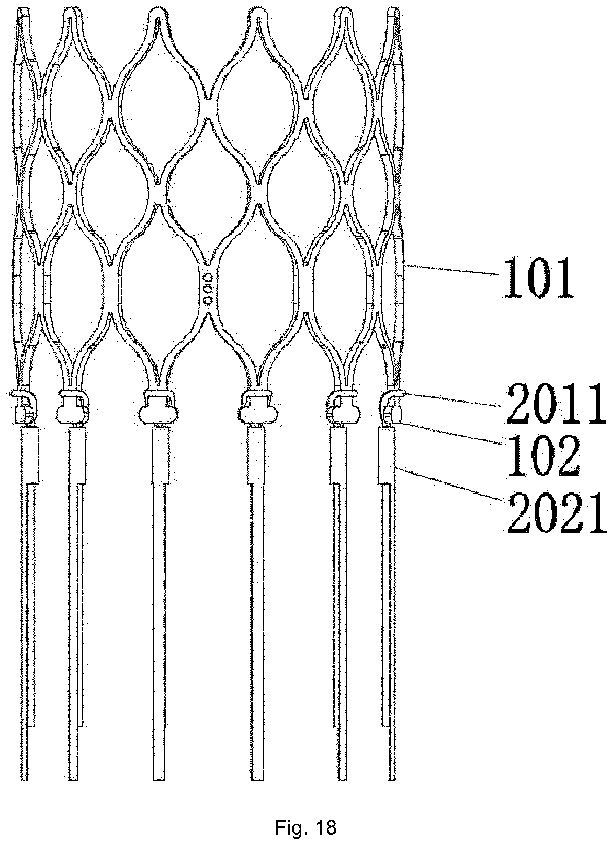

FIG. 18 is a schematic view (contracted state) of a connection form of the lasso structure and the lug structure in the present invention;

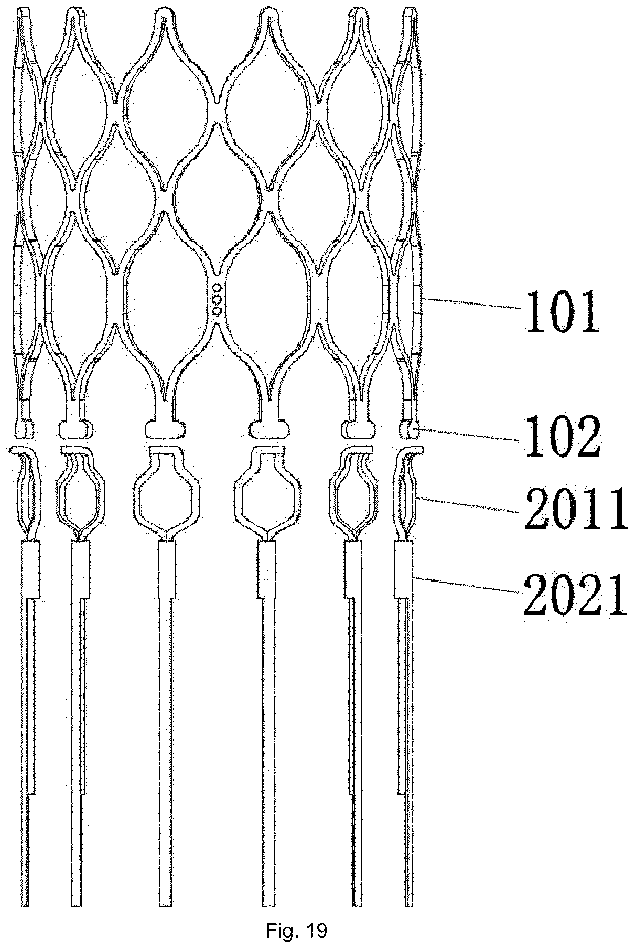

FIG. 19 is a schematic view (expanded state) of a connection form of the lasso structure and the lug structure in the present invention;
FIG. 20 is a schematic view of a valve stent partially deployed when the valve stent is implanted into a body via a transapical route in the present invention;
FIG. 21 is a schematic view of the valve stent of FIG. 20 fully deployed;
FIG. 22 is a schematic view of the unlocking sleeve system of FIG. 20 withdrawn and releasing the valve stent;
FIG. 23 is a schematic view of the connection release system of FIG. 20 detached from the valve stent;
FIG. 24 is a schematic view of the valve stent of FIG. 20 anchored on the valve annulus position;
FIG. 25 is a schematic view of a valve stent partially deployed when the valve stent is implanted into a body via a transfemoral artery route in the present invention;
FIG. 26 is a schematic view of the valve stent of FIG. 25 fully deployed;
FIG. 27 is a schematic view of the release object of the valve stent beginning after withdrawal of the unlocking sleeve system of FIG. 25;
FIG. 28 is a schematic view of the connection release system of FIG. 25 detached from the valve stent; and
FIG. 29 is a schematic view of the valve stent of FIG. 25 anchored on the valve annulus position.

Reference numerals in the drawings: 100, release object; 101, valve stent body; 102, lug structure; 104, inflow end of the valve stent; 105, outflow end of the valve stent; 200, connection release structure; 201, connection fixing system; 2011, connection fixing structure; 2012, first finger-shaped connecting rod; 2013, inner tube; 202, unlocking sleeve system; 2021, unlocking sleeve; 2022, second finger-shaped connecting rod; 2023, outer tube; 300, loading sheath tube; 401, valve annulus position; 402, original valve leaflet; 403, left ventricle; 404, cardiac apex; 406, aorta.

### DETAILED DESCRIPTION OF THE INVENTION

The present invention now will be described more clearly and fully hereinafter with reference to the drawings, and it is evident that the embodiments described are a part, not all, of the embodiments of the invention. Based on the embodiments of the present invention, all other embodiments obtained by a person of ordinary skill in the art without involving any inventive effort are within the scope of the present invention.

In describing the present invention, it is to be understood that the terms "center, "upper", "lower", "left", "right", "vertical", "horizontal", "inner", "outer", and the like, indicate orientations or positional relationships that are based on the orientations or positional relationships shown in the drawings and are merely intended to facilitate describing the present invention and to simplify the description. It is not intended to indicate or imply that the referenced device or element must have a particular orientation, be constructed and operated in a particular orientation, and thus should not be construed as limiting the invention. Furthermore, the terms "first", "second", "third" are used for descriptive purposes only and are not to be construed as indicating or implying relative importance.

In describing the present invention, it should be noted that the terms "mounted", "connected", and "connecting" are to be interpreted broadly, for example, either fixedly or removably, or integrally, mechanically connected or electrically connected, connected directly or indirectly via an intermediary, and connected between the two elements internally, unless specifically stated and defined otherwise. It will be understood by those of ordinary skill in the art that the specific meanings of the above terms in the present invention may be specifically understood.

Furthermore, the technical features involved in the different embodiments of the invention described below may be combined with each other as long as they do not constitute a conflict with each other.

As used in this disclosure and the appended claims, the singular forms "a", "an", and "the" are intended to include the plural forms as well, unless the context clearly indicates other meanings. It should also be understood that the term "and/or" as used herein refers to and includes any or all possible combinations of one or more of the associated listed items.

The word "multiple" or "a plurality of' in the present disclosure and appended claims means two or more, unless specifically stated otherwise.

### Embodiment 1

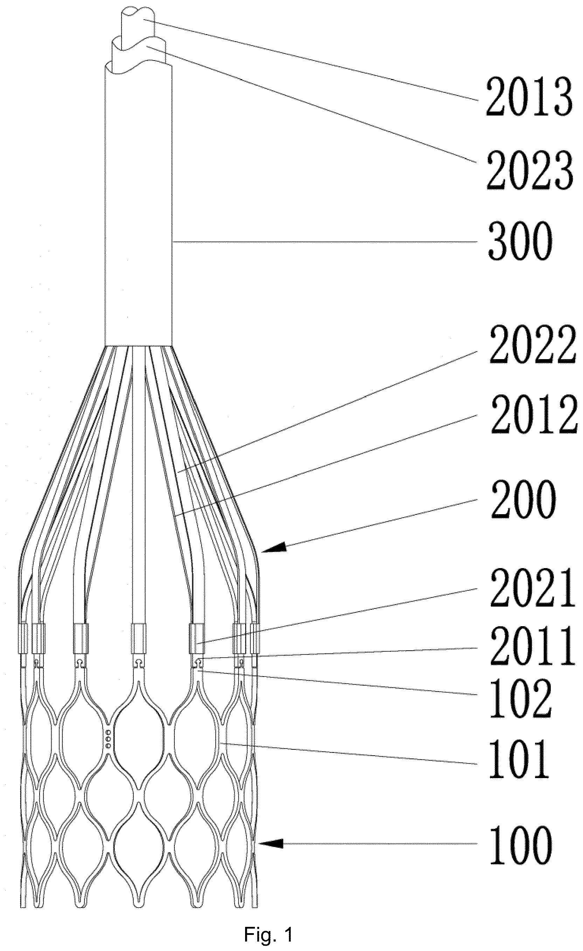

One embodiment of a connection release structure 200 is shown in FIGs. 1-2 for connecting a release object 100, and more specifically for capturing and releasing a release object 100. It includes a connection fixing system 201 and an unlocking sleeve system 202, wherein the connection fixing system 201 includes a connection fixing structure 2011 movably connected with a loading sheath tube 300, the unlocking sleeve system 202 includes an unlocking sleeve 2021 movably connected with the loading sheath tube 300, and when the connecting and releasing structure is configured for being matched with a release object, the connection fixing structure 2011 is configured for being connected with the release object 100, and the unlocking sleeve 2021 is configured for locking the connection fixing structure 2011 and the release object 100.

Although the connection fixing structure is a hook structure in the present embodiment, it may be one or more of a lasso structure, a clamp structure, or a hook structure.

In one possible embodiment, in a stowed state, the release object 100 is partially or completely retracted into the loading sheath tube 300; In one possible embodiment, in the stowed state, the connection release structure is partially or completely retracted into the loading sheath tube 300; In one possible embodiment, in the stowed state, the connection fixing system 201 and the unlocking sleeve system 202 are partially or completely retracted into the loading sheath tube 300.

By adopting the above scheme, the release object 100 can be captured by the connection fixing structure 2011 of the connection fixing system 201 at first, then the sleeve in the unlocking sleeve system 202 is moved to the joint of the connection fixing structure 2011 and the release object 100 under the action of external force, and the connection fixing structure 2011 and the release object 100 are locked to prevent the connection fixing structure 2011 and the release object 100 from being separated due to the action of external force; and the locked connection fixing structure 2011 and the release object 100 can be fixed together, and by means of a transcatheter technology, the release object 100, such as a prosthetic heart valve, can be delivered into an interventional catheter (a loading sheath tube 300) through a femoral artery to a designated area, such as a valve area of an aorta 406, to be deployed. Since the release object 100 is connected with the connection fixing system 201 and tightened by the unlocking sleeve 2021 in the unlocking sleeve system 202 in the delivering process, the locked connection fixing structure 2011 and the release object 100 can be adjusted several times without causing the connection fixing structure 2011 to be separated from the release object 100 (i.e. the release object 100 can be deployed several times so as to obtain the most appropriate deployment mode and implantation site), and improve the release accuracy of the valve stent; when the release object 100 is adjusted to an ideal position, the unlocking sleeve 2021 can be pulled by an external force to keep away from the junction, and the position of the connection fixing structure 2011 is adjusted by the external force to cause the connection fixing structure 2021 to release the release object 100, so that the release process is completed.

In one possible embodiment, the connection fixing system 201 and the unlocking sleeve system 202 are partially or completely retracted into the loading sheath tube 300 in the stowed state; and more preferably, the connection fixing structure 2011 and the unlocking sleeve 2021 are partially or completely retracted into the loading sheath tube 300 in the stowed state. Since the connection release structure 200 may be retracted into the loading sheath tube 300, even if the release object 100 cannot be deployed in right place due to various factors, the connection release structure 200 (i.e., the connection fixing structure 2011 and the unlocking sleeve 2021) can be partially or completely retracted into the loading sheath tube 300. Meanwhile, the release object 100 connected with the connection fixing structure 2011 can be changed into a contracted state and also can be partially or completely retracted into the loading sheath tube 300, so that the retrievable of the loading sheath tube 300 is no longer hindered, the loading sheath tube 300 is conveniently and completely retracted, a fault-tolerant opportunity is provided for an operator, and the safety and reliability of the implantation of the release object 100 are improved.

In one possible embodiment, the connection fixing structure is configured to release or capture the release object.

Specifically, in a first state, the connection fixing structure 2011 is configured for being connected with the release object 100, and the unlocking sleeve 2021 is sleeved on the joint of the connection fixing structure 2011 and the release object 100; in a second state, the connection fixing structure 2011 is configured to be disconnected from the release object 100. More specifically, in the second state, the unlocking sleeve 2021 is not located at the joint of the connection fixing structure 2011 and the release object 100; more precisely, in the second state, the unlocking sleeve 2021 leaves the joint of the connection fixing structure 2011 and the release object 100 and no longer locks the connection fixing structure 2011 and the release object 100, so that the connection fixing structure 2011 can be disconnected from the release object 100 under the action of external force to release the release object 100.

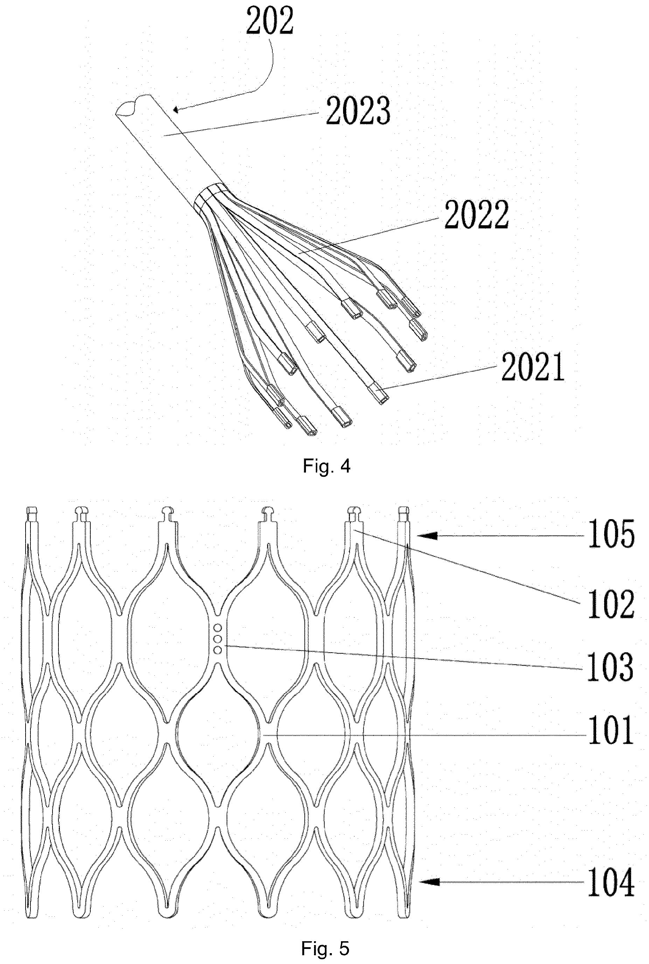

In one possible embodiment, as shown in FIG. 4, the unlocking sleeve system 202 includes an outer tube 2023 arranged on a loading sheath tube 300, the outer tube 2023 slidably connected with the loading sheath tube 300, the outer tube 2023 having a second finger-shaped connecting rod 2022 at its tail end, and the unlocking sleeve 2021 disposed on the second finger-shaped connecting rod 2022. Specifically, as shown in FIG. 4, the unlocking sleeve system 202 has an unlocking sleeve 2021, the guide portion of the unlocking sleeve system 202 is substantially the same as that of the connection fixing system 201, that is, the guide portion is composed of an outer tube 2023 and a second finger-shaped connecting rod 2022, the outer tube 2023 is sleeved outside the inner tube 2013 and may be retracted into the loading sheath tube 300, and the unlocking sleeve 2021 and the outer tube 2023 are sequentially combined by the second finger-shaped connecting rod 2022 to form a whole. The number of the second finger-shaped connecting rods 2022 is greater than or equal to the number of the first finger-shaped connecting rods 2012, and the unlocking sleeve 2021 pass through the first finger-shaped connecting rods 2012 in a one-to-one correspondence mode and are arranged on the second finger-shaped connecting rods 2022. That is, the release object can be introduced into the human body through the loading sheath tube, and the unlocking sleeve 2021 slides by applying force to one end of the outer tube away from the unlocking sleeve, so that the unlocking sleeve 2021 does not lock the connection fixing structure 2011 and the release object 100.

In one possible embodiment, in order to cause the unlocking sleeve system 202 to be partially or completely retracted into the loading sheath tube 300, the unlocking sleeve system 202 may be retracted into the loading sheath tube 300 in a manner that allows the unlocking sleeve system 202 to move relative to the loading sheath tube 300. More specifically, a second connector is provided between the second finger-shaped connecting rod 2022 and the outer tube 2023, and the second connector may be retracted into the loading sheath tube 300 in a manner that allows the unlocking sleeve system 202 to move relative to the loading sheath tube 300. For example, in some embodiments, the second connector may be configured to allow the second finger-shaped connecting rod 2022 to rotate about the connector relative to the outer tube 2023. In some embodiments, the second connector may provide a rotational connection between the second finger-shaped connecting rod 2022 and the outer tube 2023. In some embodiments, the second connector may provide a flexible attachment between the second finger-shaped connecting rod 2022 and the outer tube 2023. As described herein with reference to various embodiments of a prosthetic valve, the second connector may be of various types and configurations. For example, the second connector may include a living hinge, a flexible member, a suture, a suture wound through the opening, a pin or tab inserted through the opening, or any combination thereof. In some embodiments, the second connector is a memory metal or alloy.

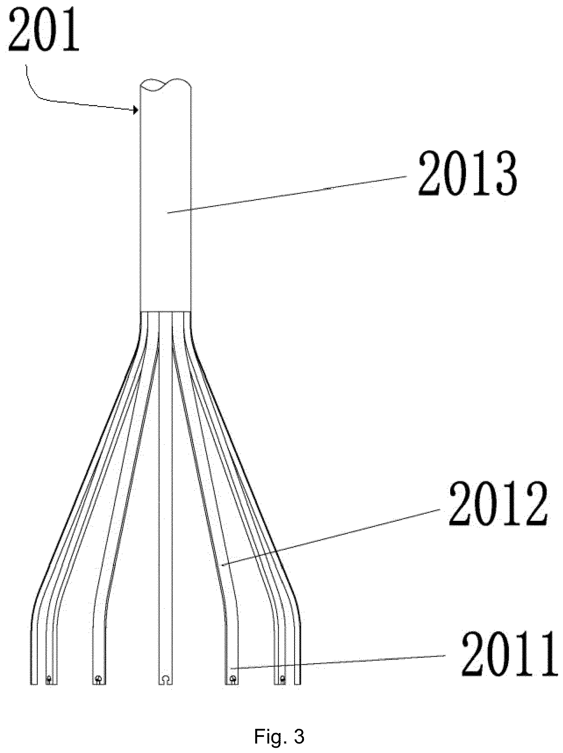

In one possible embodiment, the connection fixing system 201 includes an inner tube 2013 arranged on a loading sheath tube 300, wherein the inner tube 2013 is slidably connected with the loading sheath tube 300, the tail end of the inner tube 2013 is provided with a first finger-shaped connecting rod 2012, the connection fixing structure 2011 is arranged on the first finger-shaped connecting rod 2012 in a one-to-one correspondence mode, and the unlocking sleeve 2021 passes through the first finger-shaped connecting rod 2012 in a one-to-one correspondence mode. Specifically, the inner tube 2013 is slidably connected with the outer tube 2023, and the sliding direction of the outer tube 2023 is the same as that of the inner tube 2013. Specifically, as shown in FIG. 3, the connection fixing system 201 is a structurally schematic view, wherein the connection fixing system 201 is formed by sequentially combining a connection fixing structure 2011, a first finger-shaped connecting rod 2012 and an inner tube 2013 coaxially arranged with the loading sheath tube 300 as a whole. The connection fixing system 201 is more easily retracted into the loading sheath tube 300 when the inner tube 2013 serves as a stress gathering point, the coaxiality of the inner tube 2013 and the connection fixing structure 2011 can be ensured and subsequent transfer and release can be better completed when the connection fixing structure 2011 is connected to the inner tube 2013 by the rigid first finger-shaped connecting rod 2012, and finally the connection fixing system 201 is stably engaged with the release object 100 by the connection fixing structure 2011. In the above-described aspect, the connection fixing structure may slide by applying a force to one end of the inner tube away from the connection fixing structure, so that the connection fixing structure 2011 is disconnected from the release object 100, thereby releasing the release object.

In one possible embodiment, in order to cause the connection fixing system 201 to be partially or completely retracted into the loading sheath tube 300, the connection fixing system 201 may be retracted into the loading sheath tube 300 in a manner that allows the connection fixing system 201 to move relative to the loading sheath tube 300. More specifically, a first connector is provided between the first finger-shaped connecting rod 2012 and the inner tube 2013, and the first connector may be retracted into the loading sheath tube 300 in a manner that allows the first finger-shaped connecting rod 2012 to move relative to the loading sheath tube 300. For example, in some embodiments, the first connector may be configured to allow the first finger-shaped connecting rod 2012 to rotate about the connector relative to the inner tube 2013. In some embodiments, the first connector may provide a rotational connection between the first finger-shaped connecting rod 2012 and the inner tube 2013. In some embodiments, the connector may provide a flexible attachment between the second finger-shaped connecting rod 2022 and the inner tube 2013. As described herein with reference to various embodiments of a prosthetic valve, the first connector may be of various types and configurations. For example, the first connector may include a living hinge, a flexible member, a suture, a suture wound through the opening, a pin or tab inserted through the opening, or any combination thereof. In some embodiments, the first connector is a memory metal or alloy.

In one possible embodiment, the inner tube 2013 is slidably connected with the outer tube 2023, preferably the sliding direction of the outer tube 2023 is same as that of the inner tube 2013. More specifically, a first through hole is provided in the loading sheath tube 300, the first through hole is matched with the outer tube 2023, while a second through hole is provided in the outer tube 2023, the second through hole is matched with the inner tube 2013, and the second through hole extends in the same direction as that of the first through hole. According to the scheme, the sliding direction of the outer tube 2023 and the sliding direction of the inner tube 2013 are set to be the same direction, so that the coaxiality between the connection fixing structure 2011 and the unlocking sleeve 2021 is guaranteed in the sliding process, the stability of the valve stent is guaranteed to be controlled in the releasing process, the valve stent can be better deployed, so as to avoid the condition that one part of the valve stent is completely deployed, and the other part of the valve stent cannot be completely deployed. At the same time, since both the outer tube 2023 and the inner tube 2013 are arranged in the loading sheath tube 300, it is convenient for an operator to pull the inner tube 2013 and the outer tube 2023 to partially or completely retract the unlocking sleeve system 202 and the connection fixing system 201 into the loading sheath tube 300.

It should be understood that in some embodiments, the unlocking sleeve 2021 is buckling, rotationally, or magnetically connected with the loading sheath tube 300. In some embodiments, the unlocking sleeve system 202 includes an outer tube 2023 disposed on the loading sheath tube 300, the outer tube 2023 movably connected with the loading sheath tube 300, the outer tube 2023 having a second finger-shaped connecting rod 2022 at its tail end, and the unlocking sleeve 2021 disposed on the second finger-shaped connecting rod 2022. The outer tube 2023 is buckling, rotationally, or magnetically connected with the loading sheath tube 300. As in some embodiments, the loading sheath tube 300 is provided with a plurality of buckling structures for securing the outer tube 2023 by which a user may choose to secure the outer tube 2023 to different positions. Alternatively, in some embodiments, the loading sheath tube 300 and the outer tube 2023 are provided with matched threads, and the loading sheath tube 300 and the outer tube 2023 are rotationally connected together via the threads.

The connection fixing system 201 includes an inner tube 2013 arranged on the loading sheath tube 300, wherein the inner tube 2013 is movably connected with the loading sheath tube 300, the tail end of the inner tube 2013 is provided with a first finger-shaped connecting rod 2012, the connection fixing structure 2011 is arranged on the first finger-shaped connecting rod 2012 in a one-to-one correspondence mode, and the unlocking sleeve 2021 passes through the first finger-shaped connecting rod 2012 in a one-to-one correspondence mode. The inner tube 2023 is buckling, rotationally, or magnetically connected with the loading sheath tube 300. More precisely, in this embodiment, the inner tube 2023 is movably connected with the outer tube 2013, and the outer tube 2013 is movably connected with the loading sheath tube 300, so that the inner tube 2023 is movably connected with the loading sheath tube 300. As in some embodiments, the outer tube 2023 is provided with a plurality of buckling structures for securing the inner tube 2013 by which a user may choose to secure the inner tube 2013 to different locations.

It should be understood that in addition to the embodiment described above in which the loading sheath tube 300 is sleeved on the outer tube 2023 and the outer tube 2023 is sleeved on the inner tube 2013, other designs are possible in which the outer tube 2023 is sleeved over the loading sheath tube 300 and the inner tube 2013 is sleeved on the outer tube 2023. It is sufficient that both the inner tube 2013 and the outer tube 2023 can be movably disposed on the loading sheath tube 300.

In one of the embodiments, the unlocking sleeve 2021 is provided with a third through hole matched with the first finger-shaped connecting rod 2012, so that the unlocking sleeve 2021 may slide along the first finger-shaped connecting rod 2012. More specifically, the unlocking sleeve 2021 shown in FIG. 4 is a hollow sleeve having a cross-sectional shape of a rectangle, a circle, and an ellipse, preferably a rectangle, to better control the stability and coaxiality of transfer in the above-described solution.

In one possible embodiment, the connection fixing structure 2011 and the unlocking sleeve 2021 are equally arranged, and both are 3-18. When they are 3, the three connection fixing structures 2011 are uniformly connected at the periphery of the release object 100 to form a stable triangular structure, so as to ensure the transfer stability and coaxiality of the invention; the number of the connection fixing structure 2011 is not more than 18 in consideration of the limitation of the diameter of the femoral artery and the convenience of surgical operation; and the number of the connection fixing structure 2011 is most preferably 12 according to multiple practices of the inventor. Naturally, since the first finger-shaped connecting rod 2012 serves to connect the connection fixing structure 2011 and the inner tube 2013, the number of the first finger-shaped connecting rod 2012 and the connection fixing structure 2011 is always equal. The second finger-shaped connecting rod 2022 serves to connect the unlocking sleeve 2021 and the outer tube 2023, so that the number of the second finger-shaped connecting rod 2022, the unlocking sleeve 2021 and the connection fixing structure 2011 is always equal. The unlocking sleeve 2021 of the unlocking sleeve system 202 shown in FIG. 4 is a hollow sleeve having a cross-sectional shape of a rectangle, a circle, and an ellipse, preferably a rectangle, to better control the stability and coaxiality of transfer in the present invention.

### Embodiment 2

Unlike Embodiment 1, this embodiment provides a connection release system including a connection fixing system 201, an unlocking sleeve system 202 and a release object 100, the connection fixing system 201 includes a connection fixing structure 2011 movably connected, preferably, slidably connected with a loading sheath tube 300, and the unlocking sleeve system 202 includes an unlocking sleeve 2021. The unlocking sleeve 2021 is movably connected, preferably, slidably connected with the loading sheath tube 300; the connection fixing structure 2011 is connected with the release object 100, and the unlocking sleeve 2021 is configured for locking the connection fixing structure 2011 and the release object 100.

Although the connection fixing structure is a hook structure in the present embodiment, it may be one or more of a lasso structure, a clamp structure, or a hook structure.

In one possible embodiment, the connection fixing structure is configured to release or capture a release object.

In one possible embodiment, in a stowed state, the release object 100 is partially or completely retracted into the loading sheath tube 300; In one possible embodiment, in the stowed state, the connection release structure is partially or completely retracted into the loading sheath tube 300; In one possible embodiment, in the stowed state, the connection fixing system 201 and the unlocking sleeve system 202 are partially or completely retracted into the loading sheath tube.

In some embodiments, the unlocking sleeve 2021 is buckling, magnetically, or slidably connected with the loading sheath tube 300.

In some embodiments, the unlocking sleeve 2021 is buckling, magnetically, or slidably connected with the loading sheath tube 300.

According to a possible one of the embodiments, in a first state, the connection fixing structure 2011 is connected with the release object 100, and the unlocking sleeve 2021 is sleeved on the joint of the connection fixing structure 2011 and the release object 100; in a second state, the connection fixing structure 2011 is disconnected from the release object 100.

In one possible embodiment, in the second state, the unlocking sleeve 2021 is not located at the joint of the connection fixing structure 2011 and the release object 100, and the connection fixing structure 2011 and the release object 100 are not locked;

In one possible embodiment, in the second state, the unlocking sleeve 2021 leaves the joint of the connection fixing structure 2011 and the release object 100.

In one possible embodiment, the connection fixing system 201 and the unlocking sleeve system 202 are partially or completely retracted into the loading sheath tube 300 in the stowed state; and more preferably, the connection fixing structure 2011 and the unlocking sleeve 2021 are partially or completely retracted into the loading sheath tube 300 in the stowed state.

In one possible embodiment, the length of the unlocking sleeve 2021 is greater than or equal to the length of the joint of the connection fixing structure 2011 and the release object 100.

In one possible embodiment, the unlocking sleeve 2021 is in clearance fit with the release object 100 and the connection fixing structure 2011;

In one possible embodiment, in the first state, the unlocking sleeve 2021 is sleeved on the joint of the connection fixture 2011 and the release object 100, and the unlocking sleeve 2021 is in clearance fit with the release object 100 and the connection fixture 2011.

In one possible embodiment, the cross-section of the unlocking sleeve 2021 is rectangular, circular or elliptical.

In one possible embodiment, the release object 100 is a self-expanding valve stent including a valve stent body 101 and a lug structure 102 matched with the connection release system, the lug structure 102 being provided on at least one end of the valve stent body 101.

In one possible embodiment, the valve stent body 101 is a mesh tubular structure, the length of which is less than its diameter.

In one possible embodiment, the length of the valve stent body 101 is 18 mm to 30 mm, and the diameter of the valve stent body is 18 mm to 35 mm.

In one possible embodiment, the lug structure 102 and the connection fixing structure 2011 are arranged as a snap fastener, the connection fixing structure 2011 is Ω-shaped, L-shaped, E-shaped, C-shaped or sawtooth-shaped, and the shape of the lug structure 102 sequentially corresponds to T-shaped, L-shaped, E-shaped, C-shaped or sawtooth-shaped.

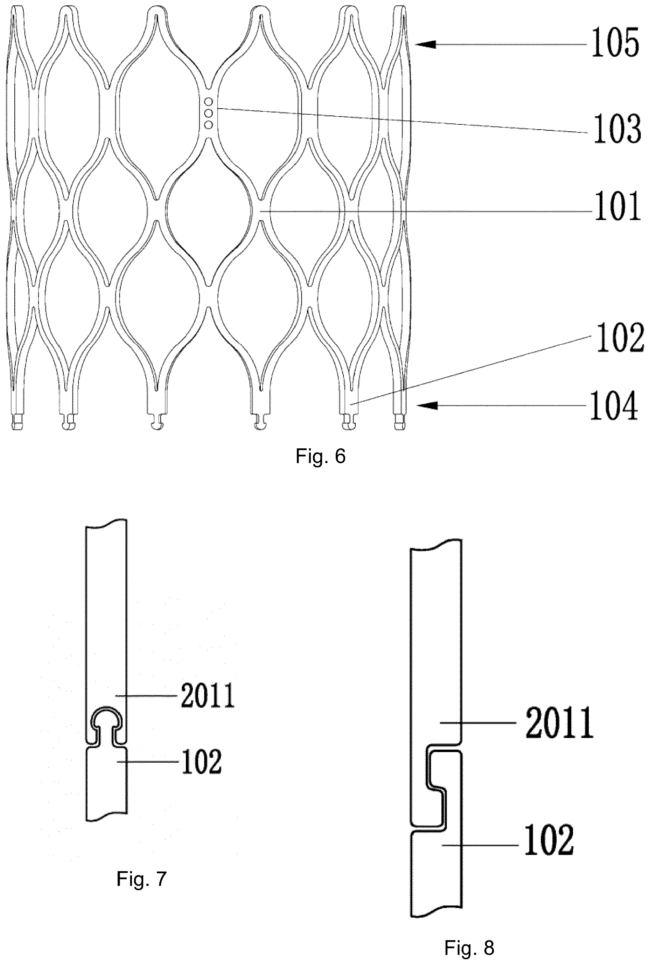

In one possible embodiment, as shown in FIG. 5, it is a structurally schematic view of the structure of the first release object 100 in this embodiment; in this embodiment, the release object 100 is preferably a valve stent, preferably a self-expanding valve stent, i.e. the valve stent has a contracted state and an expanded state, and the structure of the valve stent in the expanded state is shown in FIGs. 5 and 6. The valve stent is cut from a superelastic alloy material or a shape memory alloy material by laser cutting machine, and includes a valve stent body 101 and a lug structure 102, the valve stent body 101 has a mesh tubular structure, wherein the mesh tubular structure is formed by connecting a plurality of rhombic mesh structures, at least one end of the rhombic mesh is provided with a lug structure 102 matched with a connection release system, and the lug structure 102 is formed by extending the tail end of the rhombic mesh. In the embodiment shown in FIG. 5, the lug structure 102 of the valve stent is an extension of the mesh tubular structure at the outflow end 105 of the valve stent. Implantation via a transfemoral artery route may be accomplished by connection with the connection fixing structure 2011 of the connection release system.

In one of the embodiments, the second structure of the valve stent 100 may be as shown in FIG. 6, the lug structure 102 of the valve stent 100 is an extension of the mesh tubular structure 101 at the inflow end 104 of the valve stent, and implantation via a transapical route may be accomplished by connection with a connection fixing structure of the connection release system 200.

In one of the embodiments, the length of the valve stent is preferably 18 mm to 30 mm, the diameter of the valve stent is preferably 18 mm to 35 mm, and the length of the valve stent is less than its diameter, thereby ensuring that the valve stent used in conjunction with the connection release system does not occlude the coronary ostium after implantation and does not have a condition that causes significant conduction block.

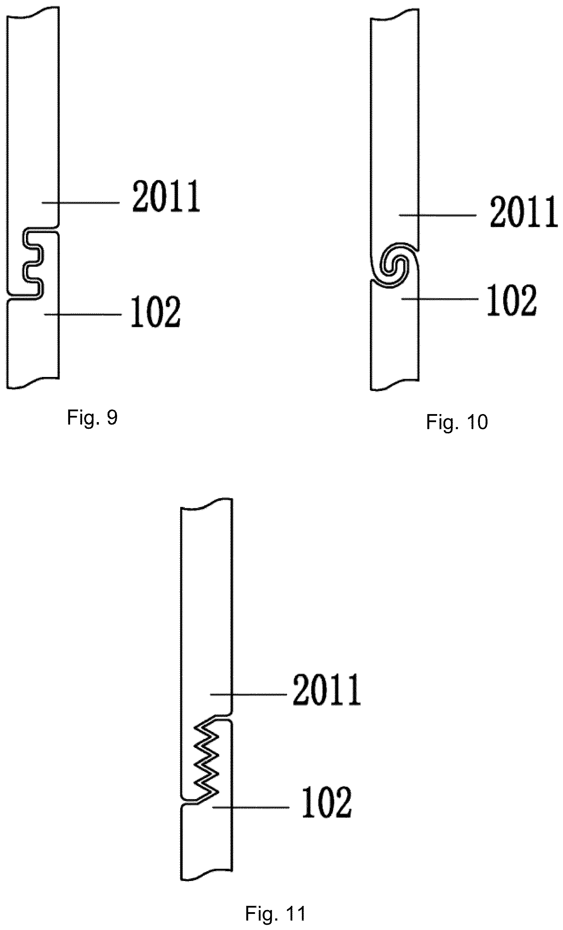

In one of the embodiments, as shown in FIGs. 7-11, the valve stent lug 102 and the connection fixing structure 2011 of the connection fixing system 201 are shown schematically in various forms of connection. A person skilled in the art would understand that in order to realize the matching and hanging connection function of the connection fixing structure 2011 and the lug structure 102, the both only need to form a whole in the form of a snap fastener, and the shapes of the both include, but are not limited to, the following contents, for example, FIG. 7 shows the connection form of the T-shaped lug structure 102 and the Ω-shaped connection fixing structure 2011, FIG. 8 shows a connection form of the L-shaped lug structure 102 and the L-shaped connection fixing structure 2011, FIG. 9 shows a connection form of the E-shaped lug structure 102 and the E-shaped connection fixing structure 2011, FIG. 10 shows a connection form of the C-shaped lug structure 102 and the C-shaped connection fixing structure 2011, and FIG. 11 shows a connection form of the sawtooth-shaped lug structure 102 and the sawtooth-shaped connection fixing structure 2011.

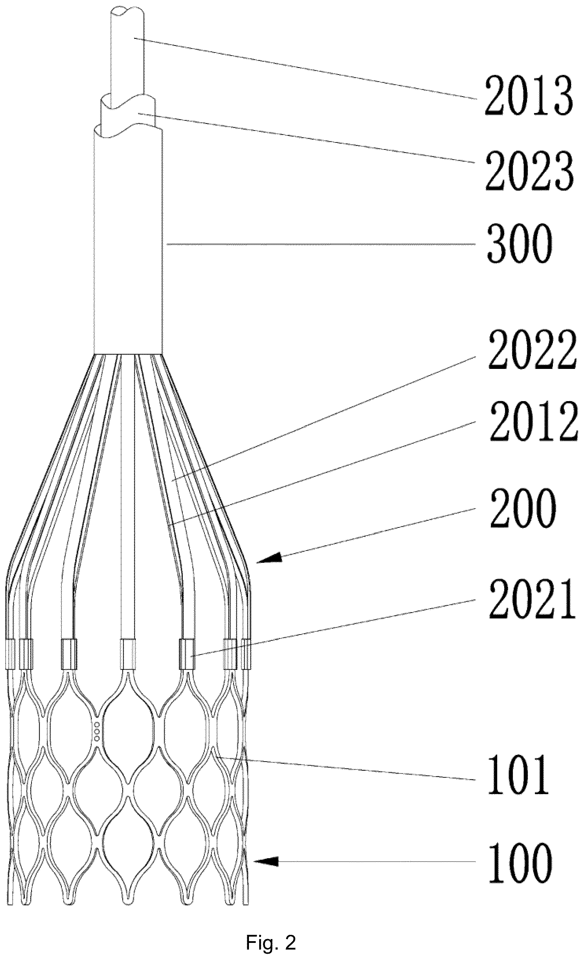

As shown in FIG. 1, it is a conversion state schematic diagram after the connection fixing structure 2011 in the connection release system 200 is connected with the lug structure 102 of the valve stent 100; and as shown in FIG. 2, it is a conversion state schematic diagram after the valve stent 100 is locked by the unlocking sleeve system 202 in the connection release system 100. The valve stent 100 and the connection release system 200 in this embodiment of the invention can be retracted into the loading sheath tube 300 in the stowed state after being connected.

In order to ensure that the unlocking sleeve 2021 covers the lug structure 102 and the connection fixing structure 2011 in the length direction, In one possible embodiment, the length of the unlocking sleeve is greater than or equal to the length of the joint of the connection fixing structure and the release. It should be understood that the size and length of the cross-sectional area of the unlocking sleeve 2021 may be adjusted and preferred according to the size and length of the cross-sectional area of the lug structure 102 and the connection fixing structure 2011 in a specific implementation.

In one possible embodiment, the unlocking sleeve 2021 is matched or in clearance fit with the release object 100 and the connection fixing structure 2011;

In one possible embodiment, in a first state, the unlocking sleeve 2021 is sleeved on the joint of the connection fixing structure 201 and the release object 100, the unlocking sleeve 2021 being matched or in clearance fit with the release object 100 and the connection fixing structure 201, more precisely, the third through hole of the unlocking sleeve 2021 being matched with the lug structure 102 and the connection fixing structure 201.

The size of the cross-sectional area of the unlocking sleeve 2021 (the third through hole) is larger than or equal to the size of the cross-sectional area of the junction formed by the hanging connection of the lug structure 102 in the valve stent 100 and the connection fixing structure 2011 in the connection fixing system 201, so that the unlocking sleeve 2021 can cover the junction of the lug structure 102 and the connection fixing structure 2011, and meanwhile, when the unlocking sleeve 2021 is positioned at the junction, the lug structure 102 and the connection fixing structure 2011 are closely matched, so that the unlocking sleeve 2021 can cover the lug structure 102 and the connection fixing structure 2011 without shaking in the cross section direction of the unlocking sleeve 2021.

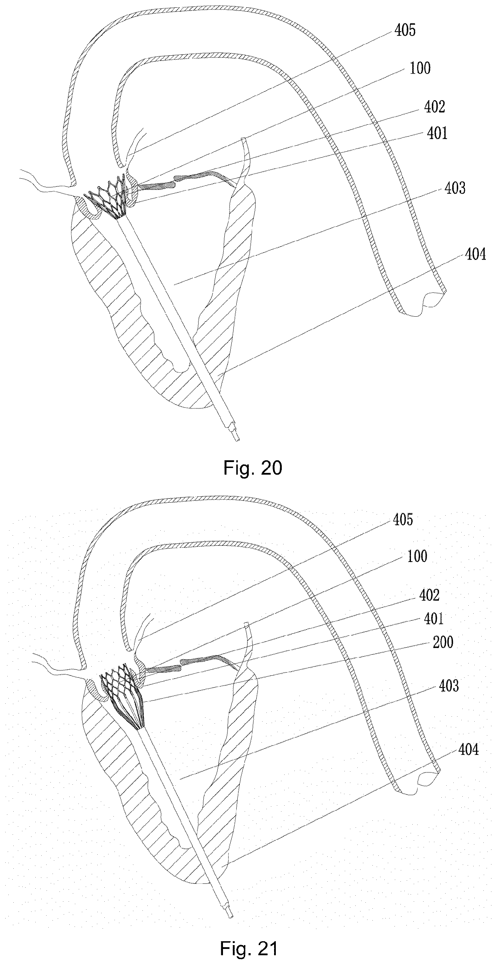

As shown in FIGs. 20-24, the present invention provides a schematic diagram of a process for implanting a valve stent and a connection release system via a transapical route:

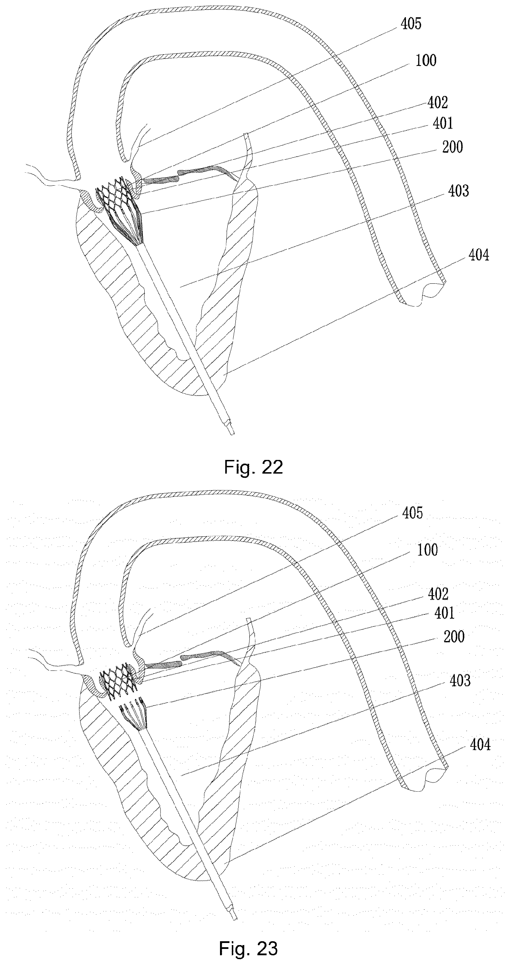

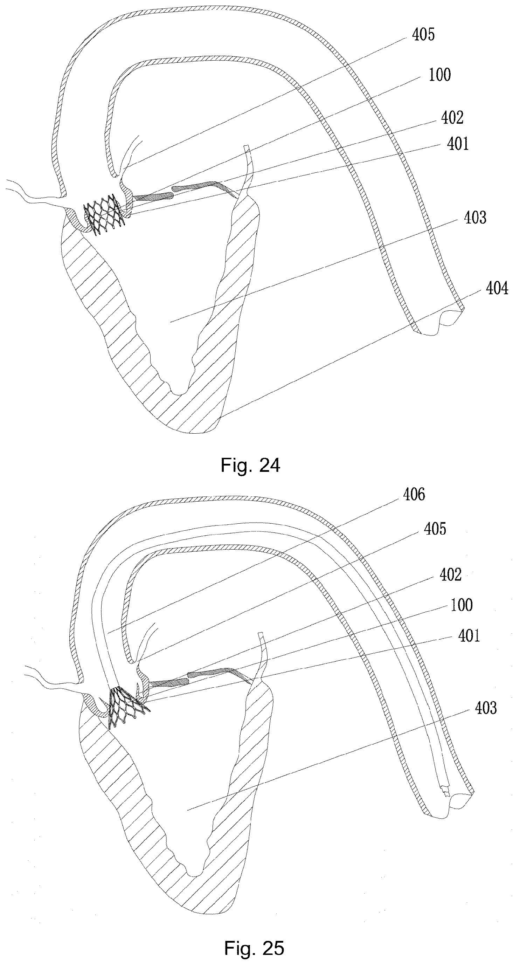

Specifically, the first valve stent 100 is first connected with the connection release system 200 and then loaded into the interior of the loading sheath tube 300, the loaded valve stent is delivered to the valve annulus position 401 by means of transcatheter technology through the left ventricle 403 by opening the cardiac apex 404, and the loading sheath tube 300 is slowly pulled back to release part of the valve stent 100, which expands the original valve leaflet 402 due to the superelasticity and shape memory properties of the valve stent material, as shown in FIG. 20. Continuing with withdrawal of the loading sheath tube 300, the valve stent 100 and the connection release system 200 are released and deployed completely, showing the expanded state, as shown in FIG. 21. At this time, the valve stent 100 completely supports the valve annulus position 401, and an operator can observe the implantation site and working condition of the prosthetic valve in the body at this time. When the operator determines that the implantation site is proper and the working condition is good, the operator moves the outer tube 2023 towards the proximal end relative to the inner tube 2013 of the connection fixing system 201 by controlling the outer tube 2023 of the unlocking sleeve system 202, and the outer tube 2023 drives the unlocking sleeve 2021 to also move towards the proximal end by the connecting rod 2022 of the unlocking sleeve, so that the lug structure 102 of the valve stent 100 and the connection fixing structure 2011 of the connection fixing system 201 are exposed; at this time, the lug structure 102 and the connection fixing system 2011 are no longer covered by the unlocking sleeve 2021, as shown in FIG. 22. The connection release system 200 and the implanted valve stent 100 are then de-loaded, and the implanted valve stent 100 anchored on the valve annulus position 401 by gently rotating or shaking the connection release system 200, as shown in FIG. 23. The connection release system 200 is then withdrawn externally from the body and the valve stent 100 is implanted as shown in FIG. 24. It will be appreciated that the proximal end is the end of the outer tube away from the unlocking sleeve.

Conversely, after the implantation process is performed, for example, as shown in FIG. 21, the operator may find the implantation site inappropriate or undesirable prior to proximal movement of the unlocking sleeve system 202. At this time, the valve stent 100 may also be re-introduced into the loading sheath tube 300 by controlling the relative movement of the connection release system 200 and the loading sheath tube 300 so as to re-adjust the implantation site or replace the valve.

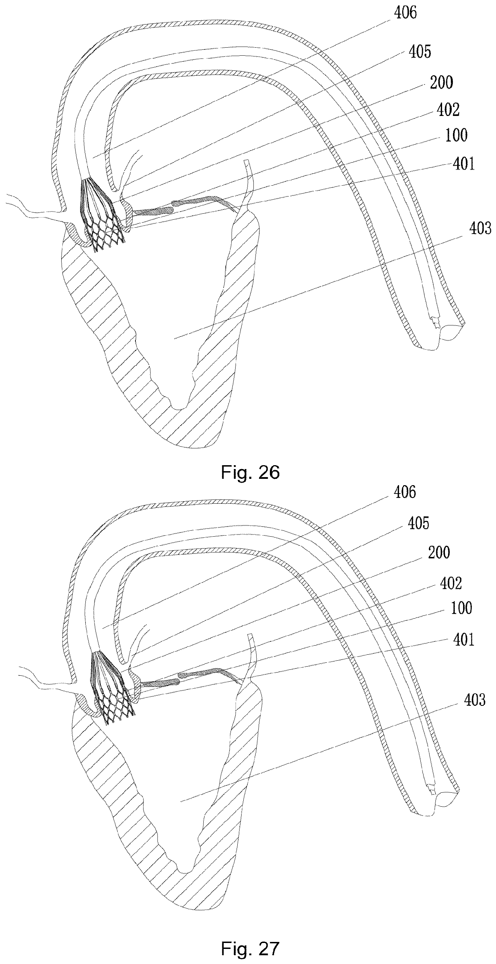

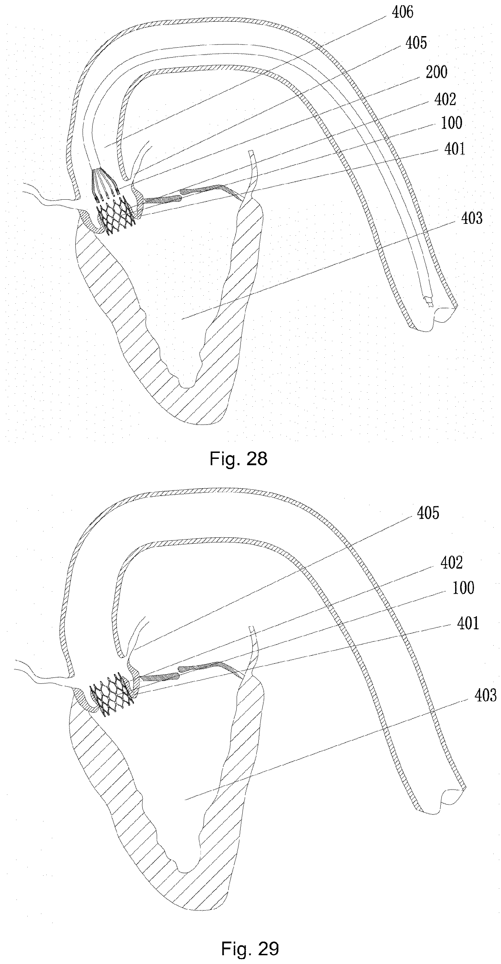

As shown in FIGs. 25-29, the present invention also provides a schematic diagram of a process for implanting a valve stent and a connection release system via a transfemoral artery route:
Specifically, the second valve stent 100 and the connection release system 200 described above are first loaded into the interior of the loading sheath tube 300, and after the loaded valve is delivered to the valve annulus position 401 via the aorta 406, the loading sheath tube 300 is slowly withdrawn backwards to release part of the valve stent 100, which expands the original valve leaflet 402 due to the superelasticity and shape memory properties of the valve stent material, as shown in FIG. 25. Continuing with withdrawal of the loading sheath tube 300, the valve stent 100 and the connection release system 200 are released and deployed completely, showing the expanded state, as shown in FIG. 26. At this time, the valve stent 100 completely supports the valve annulus position 401, and an operator can observe the implantation site and working condition of the prosthetic valve in the body at this time. When the operator determines that the implantation site is proper and the working condition is good, the operator moves the outer tube 2023 towards the proximal end relative to the inner tube 2013 of the connection fixing system 201 by controlling the outer tube 2023 of the unlocking sleeve system 202, and the outer tube 2023 drives the unlocking sleeve 2021 to also move towards the proximal end by the connecting rod 2022 of the unlocking sleeve, so that the lug structure 102 of the valve stent 100 and the connection fixing structure 2011 of the connection fixing system 201 are exposed; at this time, the lug structure 102 and the connection fixing system 2011 are no longer covered by the unlocking sleeve 2021, as shown in FIG. 27. The connection release system 200 and the implanted valve stent 100 are unloaded by gently rotating or shaking the connection release system 200, and then the implanted valve stent 100 are anchored on the valve annulus position 401, as shown in FIG. 28. The connection release system 200 is then withdrawn externally from the body and the valve stent is implanted as shown in FIG 29. Conversely, after the implantation process proceeds as shown in FIG. 26, if the operator finds that the implantation site is inappropriate or otherwise undesirable prior to proximal movement of the unlocking sleeve system 202, the operator may also re-introduce the valve stent 100 into the loading sheath tube 300 by controlling the relative movement of the connection release system 200 and loading sheath tube 300 so as to re-adjust the implantation site or replace the valve.

### Embodiment 3

In this embodiment, the connection fixing structure 2011 is a lasso structure, and hereinafter, the lasso structure will replace the connection fixing structure 2011, and it should be understood that a side of the connection fixing structure 2011 close to the loading sheath tube 300 is the proximal side of the connection fixing structure 2011 (lasso structure), and a side thereof away from the loading sheath tube 300 is the distal side of the connection fixing structure 2011 (lasso structure).

In a first state, the connection fixing structure 2011 (lasso structure) is connected with the release object 100; in a second state, the connection fixing structure 2011 (lasso structure) is disconnected from the release object 100.

In one of the embodiments, the lasso structure has a contracted state and an expanded state.

In one of the embodiments, in the first state, the lug structure 102 passes through the lasso structure, the lasso structure is in the contracted state, and the lasso structure and the lug structure 102 are mutually clamped.

In one of the embodiments, in the first state, the unlocking sleeve 2021 is close to the joint of the lasso structure and the lug structure 102, causing the lasso structure to be in the contracted state.

In one of the embodiments, in the second state, the unlocking sleeve 2021 is away from the joint of the lasso structure and the lug structure 102, causing the lasso structure to be in the expanded state.

In one of the embodiments, the distal side of the lasso structure extends radially; as the distal side of the lasso structure extends radially, the unlocking sleeve 2021, when moving toward the distal side, abuts against the distal side of the lasso structure, does not directly disengage from the lasso structure, and can change the lasso structure to the contracted state.

In one of the embodiments, the lug structure 102 is T-shaped, L-shaped, E-shaped, C-shaped, or sawtooth-shaped.

In one of the embodiments, part or all of the lasso structures have superelasticity and shape memory properties.

Specifically, initially, the lasso structure is in the expanded state, the front end portion of the lug structure 102 can pass through the lasso structure, and then an operator can move the outer tube 2023 to towards the distal end relative to the inner tube 2013 of the connection fixing system 201 by controlling the outer tube 2023 of the unlocking sleeve system 202, and the outer tube 2023 drives the unlocking sleeve 2021 to also move towards the distal end by the connecting rod of the unlocking sleeve. That is, the unlocking sleeve 2021 is brought close to the joint of the lasso structure and the lug structure 102, causing the lasso structure to be in the contracted state, and the front end portion of the lug structure 102 cannot pass back through the lasso structure, so that the lug structure 102 and the lasso structure are clamped together (that is, the first state).

When an operator finishes valve implantation and determines that the implantation site is proper and the working condition is good, the operator can move the outer tube 2023 towards the proximal end relative to the inner tube 2013 of the connection fixing system 201 by controlling the outer tube 2023 of the unlocking sleeve system 202, and the outer tube 2023 drives the unlocking sleeve 2021 to also move towards the proximal end by the connecting rod of the unlocking sleeve, namely, causing that the unlocking sleeve 2021 is away from the joint of the lasso structure and the lug structure 102; since the acting force by the unlocking sleeve 2021 is no longer applied, the reduced lasso structure expands back so that the lasso structure is in the expanded state (i.e., the second state). At this time, the front end of the lug structure 102 may pass back through the lasso structure, and then the connection release system may be gently rotated or shaken so that the connection release system and the implanted valve stent are unloaded and anchored on the valve annulus position 401.

### Embodiment 4

In order to better explain how the connection fixing structure 2011 is connected and fixed with the release object 100 when the connection fixing structure 2011 is a clamp structure, the applicant also provides the embodiment. In the embodiment, the connection fixing structure 2011 is a clamp structure; hereinafter, the connection fixing structure 2011 will be replaced with a clamp structure.

A side of the connection fixing structure 2011 close to the loading sheath tube 300 is a proximal side, and a side thereof away from the loading sheath tube 300 is a distal side;

Preferably, the clamp structure has a disconnected state and a clamped state;

Preferably, the clamp structure includes a first clamping body and a second clamping body, the proximal side of the first clamping body is fixedly connected with the proximal side of the second clamping body, and the first clamping body and the second clamping body are configured for clamping and connecting the release object 100; it should be understood that the meaning of the proximal and distal sides of the first clamping body and second clamping body is the same as the meaning of the proximal and distal sides of the connection fixing structure 2011, with the proximal side close to the loading sheath tube 300 and the distal side away from the loading sheath tube 300; preferably, when the clamp structure is in the clamped state, the distal side of the first clamping body abuts against the distal side of the second clamping body, and when the clamp structure is in the disconnected state, the distal side of the first clamping body is disconnected from the distal side of the second clamping body;

Preferably, in a first state, the first clamping body and the second clamping body clamp a part of the valve stent body 101 between the first clamping body and the second clamping body.

Preferably, in the first state, a first cavity is formed between the first clamping body and the second clamping body, and a part of the valve stent body 101 is positioned in the first cavity; it will be appreciated that the valve stent body 101 is a mesh tubular structure having a plurality of strips thereon for forming the mesh tubular structure, the first cavity being configured to mate with the strips.

Preferably, in a second state, the first clamping body is disconnected from the second clamping body;

Preferably, in the second state, the distal side of the first clamping body is separated from the distal side of the second clamping body.

In one of the embodiments, part or all of the clamp structures have superelasticity and shape memory properties, and in some embodiments, are memory metals or alloys.

Specifically, initially, the first clamping body and the second clamping body in the clamp structure are disconnected, the strips in the valve stent body 101 can be placed in the first clamping body and the second clamping body, and then an operator can move the outer tube 2023 towards the distal end relative to the inner tube 2013 of the connection fixing system 201 by controlling the outer tube 2023 of the unlocking sleeve system 202, and the outer tube 2023 drives the unlocking sleeve 2021 to also move towards the distal end by the connecting rod of the unlocking sleeve, namely, causing that the unlocking sleeve 2021 is close to the joint of the clamp structure and the valve stent body 101, so that the clamp structure is in the clamped state. The distal side of the first clamping body abuts against the distal side of the second clamping body, causing that the valve stent body 101 (a strip) is clamped between the first clamping body and the second clamping body, so that the valve stent body 101 and the connection fixing structure 2011 are clamped together (that is, the first state).

When an operator finishes valve implantation and determines that the implantation site is proper and the working condition is good, the operator can move the outer tube 2023 towards the proximal end relative to the inner tube 2013 of the connection fixing system 201 by controlling the outer tube 2023 of the unlocking sleeve system 202, and the outer tube 2023 drives the unlocking sleeve 2021 to also move towards the proximal end by the connecting rod of the unlocking sleeve, namely, causing that the unlocking sleeve 2021 is away from the joint of the clamp structure and the valve stent body 101; as the acting force of the unlocking sleeve 2021 is no longer applied, the distal side of the first clamping body no longer abuts against the distal side of the second clamping body, so that the clamp structure is in the disconnected state, that is, the distal side of the first clamping body is separated from the distal side of the second clamping body (i.e., the second state). At this time, the valve stent body 101 (a strip) is no longer clamped, and then the connection release system can be gently rotated or shaken so that the connection release system and the implanted valve stent are unloaded and anchored on the valve annulus position 401.

It should be understood that in this embodiment, the valve stent need not be provided with the lug structure 102.

### Embodiment 5

A connection release system for a medical implant, retracted into a loading sheath tube 300, includes a connection fixing system 201 for connecting the medical implant and an unlocking sleeve system 202 for locking or releasing the medical implant from the connection fixing system 201, the connection fixing system 201 and the unlocking sleeve system 202 being relatively slidably disposed in the loading sheath tube 30 from inside to outside, wherein the connection fixing system 201 is provided with a connection fixing structure 2011 for capturing the medical implant, the unlocking sleeve system 202 is provided with an unlocking sleeve 2021, and after the connection fixing structure 2011 is engaged with the medical implant, the unlocking sleeve 2021 slides to a junction to be locked under the action of external force or slides away from the junction to be released.

Although the connection fixing structure 2011 is a hook structure in the present embodiment, it may be one or more of a lasso structure, a clamp structure, or a hook structure.

In one possible embodiment, the connection fixing system 201 includes an inner tube 2013 coaxially arranged with the loading sheath tube 300, the tail end of the inner tube 2013 is provided with a first finger-shaped connecting rod 2012, and the connection fixing structure 2011 is arranged on the finger-shaped connecting rod in a one-to-one correspondence mode.

In one possible embodiment, the unlocking sleeve system 202 includes an outer tube 2023 sleeved outside the inner tube 2013, the tail end of the outer tube 2023 is provided with a second finger-shaped connecting rod 2022 which is not fewer than the first finger-shaped connecting rod 2012, and the unlocking sleeve 2021 passes through the first finger-shaped connecting rod 2012 in a one-to-one correspondence mode and is arranged on the second finger-shaped connecting rod 2022.

In one possible embodiment, the connection fixing structure 2011 and the unlocking sleeve 2021 are equally arranged, and both are 3-18.

The various technical features of the above-mentioned embodiments can be combined in any combination, and in order to simplify the description, all possible combinations of the various technical features of the above-mentioned embodiments are not described. However, as long as there is no contradiction between these combinations of technical features, they should be considered to fall in the scope of the description.

Although only specific embodiments of the present invention have been described above, the scope of the present invention is not limited thereto, and any changes or substitutions that may be readily made by those skilled in the art within the scope of the present disclosure are intended to be within the scope of the present invention. Therefore, the scope of the invention should be determined with reference to the appended claims.

## Claims

1. A connection release structure for connecting a release object, comprising a connection fixing system and an unlocking sleeve system, wherein the connection fixing system comprises a connection fixing structure movably connected with a loading sheath tube, the unlocking sleeve system comprises an unlocking sleeve movably connected with the loading sheath tube, and when the connection release structure is configured for being matched with the release object, the connection fixing structure is configured for being connected with the release object, and the unlocking sleeve is configured for fixing or unlocking the connection fixing structure and the release object.

2. The connection release structure according to claim 1, wherein the connection fixing structure may be one or more of a hook structure, a clamp structure or a lasso structure;
preferably, a side of the connection fixing structure close to the loading sheath tube is a proximal side, and a side thereof away from the loading sheath tube is a distal side;
preferably, the clamp structure has a disconnected state and a clamped state;
preferably, the clamp structure comprises a first clamping body and a second clamping body, the proximal side of the first clamping body is fixedly connected with the proximal side of the second clamping body, and the first clamping body and the second clamping body are configured for clamping and connecting the release object; preferably, when the clamp structure is in the clamped state, the distal side of the first clamping body abuts against the distal side of the second clamping body, and when the clamp structure is in the disconnected state, the distal side of the first clamping body is disconnected from the distal side of the second clamping body; and
preferably, the lasso structure has a contracted state and an expanded state.

3. The connection release structure according to claim 1 or 2, wherein the connection fixing structure is configured to release or capture the release object.

4. The connection release structure according to any one of claims 1-3, wherein, in a stowed state, the connection release structure is configured to partially or completely retract the release body into the loading sheath tube;
preferably, in the stowed state, the connection release structure is partially or completely retracted into the loading sheath tube; more preferably, in the stowed state, the connection fixing system and the unlocking sleeve system are partially or completely retracted into the loading sheath tube;
preferably, in the stowed state, the connection fixing system and the unlocking sleeve system are partially or completely retracted into the loading sheath tube; and more preferably, in the stowed state, the connection fixing structure and the unlocking sleeve are partially or completely retracted into the loading sheath tube.

5. The connection release structure according to any one of claims 1-4, wherein, in a first state, the connection fixing structure is configured for being connected with the release object; in a second state, the connection fixing structure is configured for being disconnected from the release object;
preferably, in the first state, the unlocking sleeve is configured for being sleeved at the joint of the connection fixing structure and the release object; or in the first state, the unlocking sleeve is close to the joint of the connection fixing structure and the release object;
preferably, in the second state, the unlocking sleeve is not located at the joint of the connection fixing structure and the release object; and
preferably, in the second state, the unlocking sleeve leaves the joint of the connection fixing structure and the release object.

6. The connection release structure according to claim 4, wherein, in the stowed state, the connection fixing system and the unlocking sleeve system are partially or completely retracted into the loading sheath tube; and more preferably, in the stowed state, the connection fixing structure and the unlocking sleeve are partially or completely retracted into the loading sheath tube.

7. The connection release structure according to any one of claims 1-6, wherein the unlocking sleeve system comprises an outer tube arranged on the loading sheath tube, the outer tube is movably connected with the loading sheath tube, a tail end of the outer tube is provided with a second finger-shaped connecting rod, and the unlocking sleeve is arranged on the second finger-shaped connecting rod; and
preferably, the outer tube is buckling, magnetically, rotationally and/or slidably connected, most preferably, slidably connected with the loading sheath tube.

8. The connection release structure according to any one of claims 1-7, wherein the connection fixing system comprises an inner tube arranged on the loading sheath tube, the inner tube is movably connected with the loading sheath tube, the tail end of the inner tube is provided with a first finger-shaped connecting rod, and the connection fixing structure is arranged on the first finger-shaped connecting rod in a one-to-one correspondence mode;
preferably, the clamp structure comprises a first clamping body and a second clamping body, the proximal side of the first clamping body is connected with the first finger-shaped connecting rod, and the proximal side of the second clamping body is connected with the first finger-shaped connecting rod;
preferably, in a first state, the distal side of the first clamping body abuts against the distal side of the second clamping body; preferably, in a second state, the distal side of the first clamping body is disconnected from the distal side of the second clamping body;
preferably, the inner tube is buckling, magnetically, rotationally and/or slidably connected, and further preferably, slidably connected with the loading sheath tube,;
preferably, the unlocking sleeve passes through the first finger-shaped connecting rods in a one-to-one correspondence mode;
preferably, the inner tube is slidably connected with the outer tube ;
preferably, the sliding direction of the outer tube is the same as that of the inner tube; preferably, the loading sheath tube is provided with a first through hole matched with the outer tube;
preferably, the outer tube is internally provided with a second through hole matched with the inner tube, and the extending direction of the second through hole is the same as that of the first through hole;
preferably, the first through hole and the second through hole are coaxially arranged; and
preferably, the connection fixing structure and the unlocking sleeve are equally arranged, and both are 3-18, preferably, 3-6, and most preferably, 3, or preferably, 9-15, and most preferably, 12.

9. A connection release system, comprising a connection fixing system, an unlocking sleeve system and a release object, wherein the connection fixing system comprises a connection fixing structure movably connected with a loading sheath tube, the unlocking sleeve system comprises an unlocking sleeve movably connected with the loading sheath tube, and the connection fixing structure is connected with the release object, and the unlocking sleeve locks the connection fixing structure and the release object.

10. The connection release system according to claim 9, wherein, in a stowed state, the release object is partially or completely retracted into the loading sheath tube;
preferably, in the stowed state, the connection release structure is partially or completely retracted into the loading sheath tube; more preferably, in the stowed state, the connection fixing system and the unlocking sleeve system are partially or completely retracted into the loading sheath tube.

11. The connection release system according to claim 9 or 10, wherein the connection fixing structure is configured to release or capture the release object.

12. The connection release system according to any one of claims 9-11, wherein, in the first state, the connection fixing structure is connected with the release object; in the second state, the connection fixing structure is disconnected from the release object;
preferably, the cross-section of the unlocking sleeve is rectangular, circular or elliptical.

13. The connection release system according to claim 10, wherein, in the stowed state, the connection fixing system and the unlocking sleeve system are partially or completely retracted into the loading sheath tube; and more preferably, in the stowed state, the connection fixing structure and the unlocking sleeve are partially or completely retracted into the loading sheath tube.

14. The connection release system according to any one of claims 9-13, wherein the release object is a self-expanding valve stent comprising a valve stent body;
preferably, the self-expanding valve stent further comprises a lug structure matched with the connection release system, and at least one end of the valve stent body is provided with the lug structure;
preferably, a side of the connection fixing structure close to the loading sheath tube is a proximal side, and a side thereof away from the loading sheath tube is a distal side;
preferably, the valve stent body is of a mesh tubular structure, and the length of the valve stent body is smaller than the diameter thereof;
preferably, the length of the valve stent body is 18 mm-30 mm, and the diameter of the valve stent body is 18 mm-35 mm;
preferably, part or all of the connection fixing structures are hook structures, the lug structure and the hook structure are arranged as a snap fastener, the hook structure is Ω-shaped, L-shaped, E-shaped, C-shaped or sawtooth-shaped, and the shape of the lug structure sequentially corresponds to T-shaped, L-shaped, E-shaped, C-shaped or sawtooth-shaped;
preferably, in the second state, the unlocking sleeve is not located at a joint of the hook structure and the release object;
preferably, in the second state, the unlocking sleeve leaves the joint of the hook structure and the release object;
preferably, the length of the unlocking sleeve is greater than or equal to the length of the joint of the hook structure and the release object;
preferably, the unlocking sleeve is matched or in clearance fit with the release object and the hook structure;
preferably, in the first state, the unlocking sleeve is sleeved on the joint of the connection fixing structure and the release object;
more preferably, in the first state, the unlocking sleeve is sleeved on the joint of the hook structure and the release object, and the unlocking sleeve is matched or in clearance fit with the release object and the hook structure;
also preferably, part or all of the connection fixing structures are lasso structures; preferably, the lasso structure has a contracted state and an expanded state;
preferably, in the first state, the lug structure passes through the lasso structure, the lasso structure is in the contracted state, and the lasso structure and the lug structure are mutually clamped;
preferably, in the first state, the unlocking sleeve is close to the joint of the lasso structure and the lug structure, more preferably, causing the lasso structure to be in the contracted state;
preferably, in the second state, the unlocking sleeve is away from the joint of the lasso structure and the lug structure, more preferably, causing the lasso structure to be in the expanded state;
preferably, the distal side of the lasso structure extends radially;
preferably, the lug structure is T-shaped, L-shaped, E-shaped, C-shaped or sawtooth-shaped;
also preferably, part or all of the connection fixing structures are clamp structures, the clamp structure comprises a first clamping body and a second clamping body, and the proximal side of the first clamping body is fixedly connected with the proximal side of the second clamping body;
preferably, in the first state, the first clamping body and second clamping body clamp a part of the valve stent body between the first clamping body and second clamping body;
preferably, in the first state, a first cavity is formed between the first clamping body and the second clamping body, and a part of the valve stent body is positioned in the first cavity;
preferably, in the second state, the first clamping body is disconnected from the second clamping body; and
preferably, in the second state, the distal side of the first clamping body is separated from the distal side of the second clamping body.

15. A connection release system for a medical implant retracted into a loading sheath tube, comprising a hook system for connecting the medical implant and an unlocking sleeve system for locking or releasing the hook system from the medical implant, the hook system and the unlocking sleeve system being relatively slidably disposed within the loading sheath tube from inside to outside, wherein the hook system has a hook structure for capturing the medical implant, the unlocking sleeve system is provided with an unlocking sleeve, and after the hook structure is engaged with the medical implant, the unlocking sleeve slides to a joint to be locked or slides away from the joint to be released under the action of external force;
preferably, the hook system comprises an inner tube coaxially arranged with the loading sheath tube, the tail end of the inner tube is provided with a first finger-shaped connecting rod, and the hook structure is arranged on the finger-shaped connecting rod in a one-to-one correspondence mode;
preferably, the unlocking sleeve system comprises an outer tube sleeved outside the inner tube, the tail end of the outer tube is provided with a second finger-shaped connecting rod which is not fewer than the first finger-shaped connecting rod, and the unlocking sleeve passes through the first finger-shaped connecting rod in a one-to-one correspondence mode and is arranged on the second finger-shaped connecting rod; and
preferably, the hook structure and the unlocking sleeve are equally arranged, and both are 3-18.
